# FASCICULE DE BREVET EUROPEEN

(11) **EP 3 359 554 B1**
(45) Date de publication et mention de la délivrance du brevet: **22.04.2026**
(21) Numéro de dépôt: 16778419.8
(22) Date de dépôt: 10.10.2016
(51) Int. Cl.: C07K 7/06, C07K 5/11, C07K 5/09, A61K 38/00, A61K 8/64, A61K 38/06, A61K 38/07, A61K 38/08, A61P 17/14, A61Q 7/00

(54) **PEPTIDES UTILES DANS LE TRAITEMENT PREVENTIF ET CURATIF DE L'ALOPECIE**
PEPTIDE ZUR VERWENDUNG BEI DER PRÄVENTIVEN UND KURATIVEN BEHANDLUNG VON ALOPEZIE
PEPTIDES OF USE IN THE PREVENTIVE AND CURATIVE TREATMENT OF ALOPECIA

(30) Priorité: 09.10.2015 FR 1559655
(43) Date de publication de la demande: 15.08.2018
(73) Titulaire: INSTITUT EUROPEEN DE BIOLOGIE CELLULAIRE, 31000 Toulouse (FR); Centre de Recherches Biologiques et d'Expérimentations Cutanées, 91160 Longjumeau (FR); Centre National de la Recherche Scientifique (CNRS), 75016 Paris (FR)
(72) Inventeur: HOCQUAUX, Michel, 75012 Paris (FR); ALMEIDA ÉPOUSE SCALVINO, Stéphanie, 92340 Bourg La Reine (FR); LATI, Elian, 92170 Vanves (FR); BAKALA, Joanna, 75012 Paris (FR)
(74) Mandataire: Regimbeau
(86) Numéro de dépôt international: PCT/EP2016/074238
(87) Numéro de publication internationale: WO 2017/060533

(56) Documents cités:
- EP-A1- 2 894 160
- WO-A2-00/52147
- WO-A2-2005/010027
- GB-A- 2 387 386
- US-A1- 2005 080 015
- REIMER U ET AL: "Side-chain effects on peptidyl-prolyl cis/trans isomerisation", JOURNAL OF MOLECULAR BIOLOGY, ACADEMIC PRESS, UNITED KINGDOM, vol. 279, no. 2, 5 June 1998 (1998-06-05), pages 449 - 460, XP004453970, ISSN: 0022-2836, DOI: 10.1006/JMBI.1998.1770
- J. M. FLETCHER ET AL: "Design of a Conformationally Defined and Proteolytically Stable Circular Mimetic of Brain-derived Neurotrophic Factor", JOURNAL OF BIOLOGICAL CHEMISTRY, vol. 283, no. 48, 22 September 2008 (2008-09-22), US, pages 33375 - 33383, XP055275272, ISSN: 0021-9258, DOI: 10.1074/jbc.M802789200

## Description

L'objet de la présente invention concerne une nouvelle famille de composés peptidiques ainsi que leurs procédés de synthèse, leur utilisation pour diminuer la chute des cheveux et stimuler leur pousse.

### Introduction

L'apparence est un facteur social important de notre époque. La chevelure possède une forte valeur symbolique ; elle participe largement à l'image que le sujet a de lui-même et qu'il offre au regard des autres. La perte des cheveux est un réel problème vécu par de nombreuses personnes comme un handicap. Pour cette raison la chevelure est au centre de toutes les attentions du secteur de soins capillaires qui pèse un quart du marché cosmétique mondial. Par conséquence on assiste à une recherche constante d'actifs efficaces et dépourvus d'effets secondaires permettant de supprimer ou de réduire la chute des cheveux (alopécie).

Aujourd'hui, en France, plus de 10 millions de personnes sont concernées par la chute anormale de cheveux. Elle touche pratiquement 2 hommes sur 3 et 1 femme sur 5 (généralement après l'âge de la ménopause). Les causes de la perte anormale de cheveux peuvent être diverses : vieillissement capillaire, maladies comme le cancer ou le lupus, modifications hormonales, stress, prise de certains médicaments ou carences alimentaires.

La chevelure humaine représente un ensemble d'environ 100 000 cheveux. Tous ces cheveux naissent, vivent puis meurent pour être ensuite remplacés par une nouvelle pousse. Chacun d'entre eux est produit par le follicule pileux qui est une annexe cutanée autonome avec son propre contrôle hormonal, son propre cycle, une structure complexe et stable *(*Bernard BA, « La vie révélée du follicule de cheveu humain. » Médecine/Sciences 2006;22:138-43*).* Les follicules servent de « réservoir » de cellules souches capables de donner naissance à toutes les lignées de cellules nécessaires pour reconstituer les follicules eux-mêmes, l'épiderme et les glandes sébacées.

Les cheveux, dont la durée de vie varie de 2 à 7 ans, ne poussent pas continuellement mais selon un rythme cyclique qui peut varier selon l'individu, son âge et les saisons. Le cycle pilaire est composé de trois phases : la phase anagène ou phase de croissance (qui dure de trois à cinq années), la phase catagène (qui dure une à deux semaines) qui est la période de repos, et la phase télogène où le cheveu meurt. À la suite de cette dernière phase qui aboutit à la chute du cheveu, le follicule se régénère par un processus de néo-morphogénèse à partir d'un réservoir de cellules souches et initie une nouvelle phase anagène.

Sur une chevelure normale qui se renouvelle en permanence, environ 85 % des follicules sont en phase de croissance, 2 % en phase de repos et plus de 10 % en phase de chute. Au cours d'une vie, on dénombre une vingtaine de cycles, jamais tous à la même phase (asynchrones), ce qui nous permet de garder une chevelure satisfaisante. Des agressions répétées, une mauvaise alimentation, la maladie et le stress, ainsi que la pollution, peuvent toutefois mettre en péril le cycle capillaire.

Dans les conditions physiologiques nous perdons chaque jour entre 50 et 100 cheveux, ayant atteint la phase télogène. Au-delà de ce nombre, on considère que la chute est excessive et doit être traitée. L*'alopécie* est un terme pour désigner une perte de cheveux sur tout ou sur une partie du cuir chevelu laissant la peau partiellement ou totalement nue. Elle touche environ 20% des hommes à partir de 20 ans, puis elle augmente à peu près 10% tous les 10 ans. Ainsi après 50 ans, un peu plus d'un homme sur deux présente un certain degré de calvitie. Si les chutes anormales de cheveux sont généralement l'apanage des hommes, cette affection peut également toucher certaines femmes et devenir un véritable handicap esthétique.

Congénitale, acquise, localisée, diffuse, aiguë ou encore chronique, il existe différentes formes d'alopécie, dont les causes et les mécanismes sont variés. En fonction de l'origine de la calvitie, les cheveux peuvent repousser plus ou moins facilement. Il n'existe pas de traitement passe-partout, chaque cas d'alopécie est complexe et doit faire l'objet d'un traitement approprié.

L'alopécie androgénétique est la plus courante forme de chute de cheveux et la principale cause de calvitie tant chez l'homme que chez la femme. Elle se traduit par une diminution progressive et définitive de la qualité et de la quantité des cheveux. L'alopécie androgénétique résulte d'une trop grande sensibilité des follicules pileux à la dihydrotestostérone (DHT), une hormone mâle, produite à partir de la testostérone. DHT abrège la phase de croissance capillaire (anagène), et par conséquent, le nombre de cycles possibles au follicule pileux s'écoule plus vite. Le cheveu finit donc par disparaître, on parle de "miniaturisation" du cheveu.

L'administration orale d'antiandrogènes oraux comme le Finastéride (Propecia^{®}), qui bloque la 5-alpha réductase, une enzyme responsable de la transformation de la testostérone en DHT au niveau des follicules pileux, réduit la chute des cheveux et active leur repousse. Cependant, ce produit qui était porteur de beaucoup d'espoir est contre indiqué chez la femme et de récentes mises en garde ont été annoncé quant à son utilisation chez l'homme. En effet, les résultats d'une étude clinique suggèrent qu'il existe un risque de développer une forme grave du cancer de la prostate chez les patients prenant ce médicament *(*Lynn R et al., « Therapeutic hotline. Treatment of androgenic alopecia with finasteride may result in a high grade prostate cancer in patients: fact or fiction?" Dermatol Ther. 2010;23:544*.* Lebdal S et al., High-grade prostate cancer and finasteride. BIU Int. 2010; 105:456*).*

D'autre part, le Minoxidil (Rogaine^{®}) et l'Aminexil^{®}, deux solutions topiques à appliquer sur le cuir chevelu, s'avèrent également efficaces dans le traitement de l'alopécie. Leurs propriétés antichute sont associées à la stimulation de la microcirculation sanguine qui assure la bonne santé de notre capital capillaire *(*Mecklenburg L et al., « Active hair growth (anagen) is associated with angiogenesis. » J Invest Dermatol. 2000;114:909*.* Lachgar S et al., "Minoxidil upregulates the expression of vascular endothelial growth factor in human hair dermal papilla cells. " Br J Dermatol. 1998;138:407*).*

Cependant ces deux produits ne sont utiles qu'au début de l'alopécie et leur efficacité reste limitée en raison de la reprise de la chute dès l'arrêt du traitement. De plus, l'usage journalier est vraisemblablement à l'origine d'effets secondaires indésirables relevés chez des patients l'utilisant à long terme tels que réactions cutanées localisées ou effets systémiques. D'autre part, le pourcentage de répondeurs est inférieur à 50%.

Le marché actuel de soins capillaires propose des nombreux traitements pour renforcer la fibre capillaire et ralentir la chute excessive de cheveux. Cependant, de manière générale, ils ont une efficacité insuffisante, notamment parce qu'ils ne s'attaquent bien souvent qu'à l'une des causes directement impliquées dans le phénomène de l'alopécie. Ils existent également des produits purement cosmétiques contenant des agents propres à conférer du volume à la chevelure amincie. Ces agents ne stimulent cependant pas la pousse. Ils peuvent toutefois donner l'impression d'une chevelure plus fournie en recouvrant la tige du cheveu, augmentant ainsi son diamètre. Ces produits n'offrent qu'une solution temporaire puisqu'ils disparaissent après chaque shampooing.

Ainsi, les recherches ciblées ayant pour objectif l'identification des nouveaux composés permettant de supprimer ou de réduire l'alopécie sont dans la ligne de mire des chercheurs. Une des voies, souvent explorée, est la recherche des nouvelles molécules capables d'influencer l'expression des facteurs de croissance qui gèrent la pousse des cheveux.

En effet, l'homéostasie du follicule pileux et la pousse des cheveux restent sous contrôle de nombreux facteurs de croissance. C'est pourquoi un procédé pratiqué dans les cabinets médicaux qui consiste à injecter des extraits plaquettaires autologues dans le cuir chevelu permet une amélioration de l'état de la chevelure *(*Li et al., « Autologous platelet-rich plasma: a potential therapeutic tool for promoting hair growth. » Dermatol Surg. 2012;38:1040*).* Les extraits plaquettaires, par leur richesse en facteurs de croissance (VEGF, PDGF, bFGF et HGF), régénèrent, ralentissent la chute et favorisent la repousse des cheveux.

Parmi les facteurs qui participent dans la régulation du cycle pilaire figure avant tout le facteur de croissance dérivé des plaquettes (PDGF, *Platelet-Derived Growth Factor*). Le PDGF est une cytokine qui joue un rôle primordial dans la pousse des cheveux et dans l'activation de la cellule souche folliculaire *(*Karlsson et al., « Roles for PDGF-A and sonic hedgehog in development of mesenchymal components of the hair follicle. » Development. 1999;126:2611*.* Tomita et al., "PDGF isoforms induce and maintain anagen phase of murine hair follicles." J Dermatol Sci. 2006;43:105*).*

En effet, la prolifération des cellules folliculaires induite par PDGF est 100 fois plus rapide que celle observée pour d'autres cibles cellulaires de ce facteur. Il a été démontré que la production de PDGF par des précurseurs adipocytaires présents à la base des follicules pileux est indispensable dans les conditions physiologiques pour initier la phase anagène *(*Festa et al., « Adipocyte lineage cells contribute to the skin stem cell niche to drive hair cycling. » Cell. 2011;146:761*).* En effet, lorsque les cheveux meurent, la couche de graisse dans le cuir chevelu rétrécit. Le développement de tissu adipeux produisant du PDGF s'est révélé nécessaire à la régénération des cheveux.

Une étude récente a mis en évidence le rôle de l'épiderme dans la sécrétion des facteurs responsables de l'adipogenèse : IGF1, BMP2 et BMP6. Ces facteurs en stimulant la croissance des adipocytes, induisent la production du PDGF qui agit à son tour sur le follicule pour activer la pousse des cheveux *(*Donati et al., « Epidermal Wnt/β-catenin signaling regulates adipocyte differentiation via secretion of adipogenic factors. » Proc Natl Acad Sci U S A. 2014;111:E1501*).* Il y a donc une synchronisation physiologique globale entre le tissu adipeux et le follicule, avec un signal initiateur qui provient de l'épiderme.

Cette découverte de la source de signaux qui déclenchent la croissance des cheveux peut conduire au développement de nouveaux actifs pour le traitement de la calvitie. D'autre part, des composés ayant un effet stimulateur sur la production du PDGF sont des candidats potentiels pour des recherches approfondies dans le domaine d'alopécie.

Le rôle du facteur de croissance de l'endothélium vasculaire (VEGF, *Vascular Endothelial Growth Factor*) dans l'homéostasie du cuir chevelu est également largement décrit. En effet, il est bien connu qu'une bonne irrigation sanguine du cuir chevelu est essentielle pour préserver le capital pileux et que la longueur de la phase anagène des cheveux dépend de la présence du VEGF.

Il a été montré que le VEGF, un puissant facteur proangiogénique, stimule la formation du réseau vasculaire au niveau du follicule pileux et alimentera la racine durant toute la phase anagène. Ceci est déterminant pour le bon déroulement du cycle pilaire *(*Yano et al., « Control of hair growth and follicle size by VEGF-mediated angiogenesis. » J Clin Invest. 2001;107:409*).* La mise en évidence de production locale du VEGF par les cellules de la papille dermique corrobore la contribution de cette cytokine à la croissance des cheveux *(*Lachgar et al., « Vascular endothelial growth factor is an autocrine growth factor for hair dermal papilla cells., Vascular endothelial growth factor is an autocrine growth factor for hair dermal papilla cells. » J Invest Dermatol. 1996;106:17-23*).* Une forte diminution du taux de VEGF chez les personnes qui perdent leurs cheveux confirme l'importance de cette cytokine dans la conservation de notre potentiel capillaire *(*Goldman et al., « Loss of vascular endothelial growth factor in human alopecia hair follicles. » J. Invest Dermatol. 1995;104:18*).*

C'est pourquoi, une stimulation de la production du VEGF au niveau folliculaire est une de voies *prometteuses* pour stimuler la croissance des cheveux et assurer leur bonne santé *(*Gnann et al., « Hematological and hepatic effects of vascular epidermal growth factor (VEGF) used to stimulate hair growth in an animal model. » BMC Dermatology 2013;13:15*).*

Le facteur de croissance de Hepatocyte (HGF, *Hepatocyte Growth Factor*) est une cytokine multifonctionnelle impliquée également dans le contrôle de l'activité folliculaire. L'effet stimulateur de HGF sur la croissance de cheveux est largement demontré *(*Jindo et al., « Hepatocyte growth factor scatter factor stimulates hair growth of mouse vibrissae in organ culture. » J Invest Dermatol. 1994;103(3):306*.* Shimaoka et al., "Hepatocyte growth factor/scatter factor expressed in follicular papilla cells stimulates human hair growth in vitro." J Cell Physiol. 1995;165:333*.* Lee et al., "Hepatocyte growth factor (HGF) activator expressed in hair follicles is involved in in vitro HGF-dependent hair follicle elongation." J Dermatol Sci. 2001;25:156*).* De plus, le HGF induit l'expression du VEGF par les kératinocytes et de cette manière contribue au processus de l'angiogenèse *(*Gille et al., « Hepatocyte Growth Factor/Scatter Factor (HGF/SF) Induces Vascular Permeability Factor (VPF/VEGF) Expression by Cultured Keratinocytes. » J Invest Dermatol. 1998;11:1160*.* Gerristen et al., HGF and VEGF : a dynamic duo. Circulation Res. 2005;96:272*).*

D'autre part, de la même façon que pour VEGF, HGF est produit localement par les cellules de la papille dermique *(*Shimaoka et al., « Dermal papilla cells express hepatocyte growth factor. » J Dermatol Sci. 1994;7 Suppl:S79*).*

Le facteur de croissance des kératinocytes (KGF/FGF7, *Keratinocyte Growth Factor*) appartient également à la famille de cytokines qui veille sur la croissance normale des cheveux. KGF intervient directement dans la fabrication des kératinocytes par la matrice et renforce la cohésion de la kératine en stimulant ainsi la pousse de cheveux dont la partie majeure est constituée de kératinocytes. *(*Danilenko et al., "Keratinocyte growth factor is an important endogenous mediator of hair follicle growth, development and differentiation: normalization of the nu/nu follicular differentiation defect and amelioration of chemotherapy-induced alopecia. " Am J Pathol. 1995;147(1):145*.* Guo et al., "Keratinocyte growth factor is required for hair development but not for wound healing." Genes Dev. 1996;10:165*).*

Une multitude d'autres facteurs de croissance est également impliquée dans le contrôle du cycle pilaire. Parmi eux il faut citer entre autres le facteur de croissance des cellules souches (SCF ou kit-ligand, *Stem Cell Factor*) *(*Randall et al., « Stem cell factor/c-Kit signalling in normal and androgenetic alopecia hair follicles. » J Endocrinol. 2008;197:11*),* le factor de croissance des nerfs (NGF, *Nerve Growth Factor) (*Zhou et al., "Dynamic changes in nerve growth factor and substance P in the murine hair cycle induce by depilation." Dermatol. 2006;33:833*),* ainsi que le facteur de croissance 1 ressemblant à l'insuline (IGF1, *Insulin Growth Factor 1)* (*(*Ahn et al., Effect of IGF-1 on hair growth is related to the anti-apoptotic effect of IGF-1 and up-regulation of PDGF-A and PDGF-B. Ann Dermatol. 2012;24:26*.* Su et al., Insulin-like growth factor 1 and hair growth. Dermatol Online J. 1999;5:1*.* Li et al., "Exogenous IGF-1 promotes hair growth by stimulating cell proliferation and down regulating TGF-B1 in C57BL/6 mice in vivo." Growth Horm IGF Res. 2014;24:89*).*

Il faut noter qu'une augmentation de la synthèse au moins d'un des facteurs décrits ci-dessus suite au traitement avec un actif devrait se traduire par une amélioration de la croissance des cheveux.

L'alopécie est décrite comme un phénomène "polygénique" parce que la modulation de plusieurs gènes est impliquée dans la chute des cheveux. D'autre part, les prédispositions génétiques sont reconnues comme un des facteurs qui influencent l'apparition de la calvitie (van der Steen P, Traupe H, Happle R, Boezeman J, Strater R, Hamm H. « The genetic risk for alopecia areata in first degree relatives of severely affected patients. An estimate. » Acta Derm Venereol. 1992;72:373). En effet, un tiers des hommes sont atteints de calvitie avant l'âge de 45 ans, un phénomène héréditaire dépendant d'une conjonction de facteurs génétiques.

Plusieurs documents divulguent des peptides contenant au maximum 5 acides aminés, et débutant par un atome d'hydrogène, notamment les peptides H-ARPAK-OH (voir EP 2 894 160 A1, US 2005/080015 et GB 2 387 386), H-RPAK-OH (EP 2 894 160 A1), H-ARPA(D-)K-NH2 (WO 00/52147). Reimer et al. (Journal of Biological Chemistry, 283, 48, 2008, pp. 33375-33383) ont également décrit le peptide Ac-AAPAAKK-NH2. Cependant, celui-ci contient plus de 5 acides aminés, débutant par un groupement acétyl (-CO-CH3). Cependant, aucun de ces documents ne divulgue l'utilisation de tels composés pour le traitement de l'alopécie.

Cette invention décrit un effet stimulateur d'une nouvelle famille des peptides sur la croissance des cheveux maintenus en survie *ex vivo.* Ces données associées aux effets inducteurs de ces peptides sur la prolifération des cellules faisant partie du follicule pileux humain ainsi que sur la production des facteurs de croissance impliqués dans le cycle pilaire nous amènent à proposer une préparation, contenant au moins un de ces peptides qui permettrait de supprimer ou de réduire l'alopécie, et notamment d'induire ou de stimuler la croissance des cheveux et/ou d'augmenter leur densité et/ou de diminuer leur chute.

### Résumé de l'invention

L'objet de la présente invention concerne ainsi un conjugué peptidique de formule (I) :

A-X₁-X₂-Pro-Ala-X-B (I)

dans lequel:
- A représente un groupement acyle en C₆ à C₂₀ ou un résidu cholestérol;
- X₁ représente une liaison covalente, une alanine ou une proline ;
- X₂ représente une arginine, une lysine, ou une alanine ;
- X représente une lysine, une alanine, ou une phénylalanine ; et
- B représente un hydroxyle ou une amine ;
ou l'un de ses sels, préférentiellement pharmaceutiquement, dermatologiquement ou cosmétiquement acceptables.

L'objet de la présente invention concerne en outre un procédé de fabrication du conjugué peptidique de formule (I) ci-dessus, caractérisé en ce que ledit procédé comprend les étapes successives suivantes :
a. synthèse, au moins partiellement sur support solide, du brin peptidique de formule H-X₁-X₂-Pro-Ala-X-B, dans lequel
   - X₁, X₂, X sont tels que définis ci-dessus et (dont les groupements réactifs) sont convenablement protégés ;
   - B est le support solide ou un groupement OH ou NH₂ convenablement protégé ;
b. greffage du groupement A au brin peptidique de l'étape (a) par réaction d'acylation (quand il est souhaité que A ne soit pas un atome d'hydrogène), préférentiellement par l'utilisation d'un acyle activé en présence d'une base;
c. déprotection des acides aminés protégés éventuellement conjointement au clivage du peptide de la résine, lorsque B est un support solide;
d. éventuelle purification du conjugué peptidique de formule (I) obtenu, par exemple par HPLC ; et
e. récupération du produit de formule (I).

De plus, l'objet de la présente invention concerne une utilisation cosmétique d'un conjugué peptidique de formule (I) tel que défini présentement pour diminuer la chute des cheveux et/ou stimuler leur pousse.

Un autre objet de la présente invention concerne une méthode de traitement pharmaceutique, cosmétique ou dermatologique pour lutter contre l'alopécie, et/ou pour stimuler la pousse des cheveux, comprenant l'administration à un patient d'un conjugué peptidique de formule (I) tel que défini présentement.

Plus particulièrement, l'objet de la présente invention concerne une méthode de traitement telle que ci-dessus caractérisée en ce que l'administration se fait par l'application sur le cuir chevelu d'une composition comprenant au moins un peptide décrit ci-dessus. De manière avantageuse, l'objet de la présente invention concerne une méthode de traitement telle que ci-dessus caractérisée en ce qu'au moins un peptide décrit ci-dessus est associé à un autre principe actif dermatologique, par exemple améliorant l'activité sur la repousse capillaire et ayant été décrits pour cette activité.

Parmi ces composés, on peut citer :
- le Minoxidil,
- des esters de l'acide nicotinique,
- des inhibiteurs de 5α-réductase, ou encore
- un autre peptide à activité dermatologique.

En particulier, il peut être cité comme exemple de peptides à activités dermatologiques pouvant être associés aux peptides selon la présente invention, tels que ceux trouvés dans la demande PCT WO97/18239 ou encore WO2005/009456 pour ces modes de réalisations particuliers.

L'objet de la présente invention concerne également un conjugué peptidique de formule (I) tel que défini présentement, en tant que médicament.

L'objet de la présente invention concerne ainsi un conjugué peptidique de formule (I) tel que défini présentement, pour son utilisation dermatologique éventuellement en association avec un autre principe actif préférentiellement dermatologique, tels que ceux améliorant l'activité sur la repousse et ayant été décrits pour cette activité, plus particulièrement ceux cités ci-dessus (Minoxidil, esters de l'acide nicotinique, inhibiteurs de 5α-réductase et autre peptides à activité dermatologique tels que ceux trouvés dans la demande PCT WO97/18239 ou encore WO2005/009456).

De manière également avantageuse, la présente invention concerne un conjugué peptidique de formule (I) tel que défini présentement, pour son utilisation dermatologique, en tant que seul principe actif.

La présente invention concerne en outre un conjugué peptidique de formule (I) tel que défini présentement, pour son utilisation dans le traitement ou la prévention de l'alopécie.

De manière complémentaire, l'objet de la présente invention concerne donc une composition pharmaceutique, dermatologique ou cosmétique comprenant au moins un conjugué peptidique de formule (I) tel que défini présentement.

### Définitions

### « Conjugué peptidique »/ « Brin peptidique »

Le terme « peptide » (équivalent au terme « oligopeptide ») doit être compris comme des polymères d'acides aminés, lesdits acides aminés étant reliés entre eux par une liaison peptidique. Une liaison peptidique est une liaison amide liant deux acides aminés. Un peptide contient généralement entre 2 et 80 à 100 acides aminés, la limite supérieure n'étant pas clairement définie, mais concerne de manière générale le domaine des protéines. Ainsi, un « dipeptide » est un fragment polymère de deux acides aminés reliés entre eux par une liaison peptidique. Un tripeptide est un fragment polymère de trois acides aminés reliés entre eux par une liaison peptidique, etc.

Dans le cadre de la présente demande, les peptides ou conjugués peptidiques sont des tetrapeptides (c'est-à-dire composés de 4 acides aminés) ou des pentapeptides (composés de 5 acides aminés).

Lorsqu'il est fait référence à l'expression « *conjugué peptidique », « brin peptidique »* ou encore « *fragment peptidique* » ces expressions font simplement référence à la succession des acides aminés considérés.

De manière classique, les peptides (ou protéines) peuvent être extraites d'un milieu biologique ou fabriquées de manière synthétique. De manière préférée, les peptides selon la présente invention sont synthétisés. Les techniques de synthèse peptidique sont décrites dans Paul Lloyd-Williams, Fernando Albericio, Ernest Giralt, "Chemical Approaches to the Synthesis of Peptides and Proteins", CRC Press, 1997 ou Houben-Weyl, "Methods of Organic Chemistry, Synthesis of Peptides and Peptidomimetics", Vol E 22a, Vol E 22b, Vol E 22c, Vol E 22d., M. Goodmann Ed., Georg Thieme Verlag, 2002. La synthèse des peptides peut être faite en milieu liquide ou sur support solide. Les deux techniques sont applicables à la présente invention. Néanmoins pour des questions pratiques, la synthèse sur support solide est préférée selon la présente invention.

Les dénominations typiquement utilisées dans le domaine de la synthèse peptidique sont applicables en l'espèce. Par exemple, les termes « Ala » ou « A » correspondent à l'alanine, « Pro » ou « P » à la proline, « Arg » ou « R » à l'arginine, « Lys » ou « K » à la lysine, « Phe » ou « F » à la phénylalanine, etc.

Les acides aminés impliqués dans le conjugué peptidique de formule (I) selon la présente invention peuvent être de configuration L ou D, selon la stéréochimie de leur carbone asymétrique. La forme « L » exclusivement est préférée, les acides aminés naturels étant de cette forme. En outre, il peut être utilisé des mélanges de formes L et D d'un même acide aminé pour la synthèse d'un conjugué peptidique de formule (I) selon la présente invention. Le mélange racémique 50/50% (±10%) en masse est désigné « L/D ». Dans le cas des mélanges d'acides aminés, le mélange L/D est préféré, car facilement obtenu. Néanmoins, l'objet de la présente invention concerne également un peptide de formule (I) ci-dessus obtenu par l'utilisation de tout type de mélange de forme « L » ou « D » d'acide aminé concerné.

De plus, selon la présente invention, le terme « chaîne latérale d'un acide aminé » représente le fragment porté par le carbone α d'un acide aminé. Par exemple, les chaînes latérales d'acides aminés naturels tels que la glycine, la valine, l'alanine et l'acide aspartique correspondent à l'atome d'hydrogène, aux groupes isopropyle, méthyle et CH₂COOH respectivement.

Les chaînes latérales des acides aminés peuvent être protégées par des groupements protecteurs (P) et plus particulièrement N-protecteurs ou O-protecteurs lorsque ces chaînes contiennent les hétéroatomes correspondants.

Les groupements protecteurs (P) sont des groupements connus de l'homme du métier. Ces groupements protecteurs et leur utilisation sont décrits dans des ouvrages tels que par exemple Greene, "Protective Groups in Organic Synthesis", Wiley, New York, 2007 4ème édition ; Harrison et al. "Compendium of Synthetic Organic Methods", Vol. 1 à 8 (J. Wiley & sons, 1971 to 1996); Paul Lloyd-Williams, Fernando Albericio, Ernest Giralt, "Chemical Approaches to the Synthesis of Peptides and Proteins", CRC Press, 1997 ou Houben-Weyl, "Methods of Organic Chemistry, Synthesis of Peptides and Peptidomimetics", Vol E 22a, Vol E 22b, Vol E 22c, Vol E 22d., M. Goodmann Ed., Georg Thieme Verlag, 2002. Selon que ces groupements protecteurs sont portés par un atome d'azote, ils seront désignés en tant que groupements N-protecteurs. Il en est de même pour les groupements O-protecteurs, etc. Par exemple, un hydroxy peut être protégé par un groupement trityl, ou un acide carboxylique peut être protégé sous forme d'un ester tert-butylique. Si on fait une synthèse sur support solide, c'est la résine qui sert de groupement protecteur à la fonction carboxylique C-terminale.

La protection du groupe amino de l'acide aminé peut être effectuée par exemple par un groupe tert-butyloxycarbonyle (désigné ci-après par Boc-) ou un groupe -9-fluorénylméthyloxycarbonyle (désigné ci-après par Fmoc) représenté par la formule :

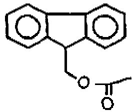

En particulier, la stratégie dite « Fmoc/tBu » est préférée due à sa facilité de mise ne œuvre. Par « tBu » il est compris que les chaînes latérales sont protégées par des groupements acido-labiles tel que tBu utilisée le plus communément dans cette stratégie. D'autre groupements protecteurs de fonctions portées par les chaînes latérales peuvent ainsi être le groupement « Boc » (pour la lysine par exemple) ou encore « Pbf » (pour l'arginine par exemple). Ainsi, la protection est effectuée selon les procédés connus de l'art antérieur. Par exemple, la protection par le groupe Boc- peut être obtenue en faisant réagir l'acide aminé avec le di-tert-butylpyrocarbonate (Boc₂O).

Comme indiqué *supra,* A peut-être un hydrogène, un groupement acyle en C6 à C20 ou un résidu cholestérol.

Lorsque A est un groupement acyle, il s'agit d'un groupement acyle en C6 à C20 linéaire, particulièrement un groupement acyle C10 à C16 linéaire, et plus particulièrement encore un groupement acyle C16 linéaire, c'est-à-dire un groupement palmitoyle.

Dans le cadre de la présente invention, le terme acyle correspond à un radical ou un groupe fonctionnel obtenu en enlevant le groupement hydroxyle d'un acide carboxylique. Le groupement acyle correspondant à un acide carboxylique de formule RCOOH aura pour formule RCO-, où l'atome de carbone et celui d'oxygène sont liés par une double liaison (groupement carbonyle). Un groupement acyle C6 à C20 correspond donc à une chaîne hydrocarbonée saturée, linéaire ou ramifiée, préférentiellement linéaire, comportant de 6 à 20 atomes de carbone dont un groupement « -C=O-, » reliant cette chaîne hydrocarbonée à la molécule qui la porte.

A peut aussi être un résidu cholestérol.

Le cholestérol est un lipide de la famille des stérols qui est impliqué dans de nombreux processus biochimiques.

La formule du cholestérol est :

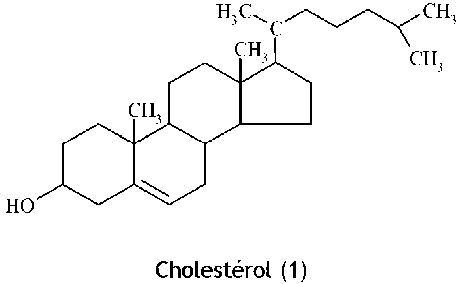

Néanmoins plusieurs centres chiraux sont présents dans cette molécule, et de manière préférée, le cholestérol utilisé pour la présente invention est :

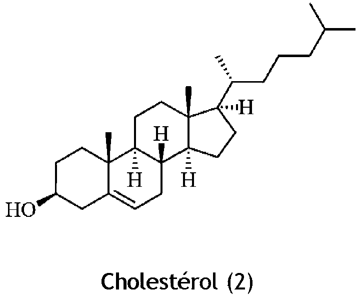

Ainsi, selon la présente invention, par « résidu cholestérol », il est compris que le groupement OH de la molécule de cholestérol est engagé dans une liaison ester avec le groupe X1.

Le terme « hydroxyle » selon la présente invention concerne le fragment -OH, et éventuellement ses sels, par exemple de sodium ou potassium.

### Description détaillée

L'objet de la présente invention concerne ainsi un conjugué peptidique de formule (I) tel que défini présentement, caractérisé en ce que le groupement A représente un groupement acyle en C₁₀ à C₂₀, préférentiellement un groupement un groupement acyle C16 palmitoyle ou un résidu cholestérol selon la formule (II) : 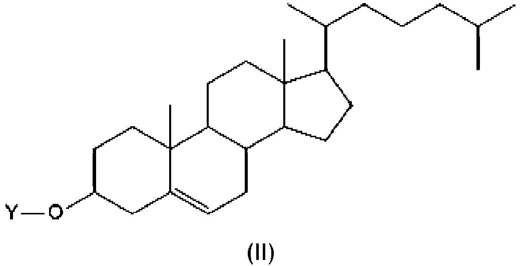 dans laquelle :
- Y représente l'atome de carbone de la liaison ester entre le résidu cholestérol et X1.

En outre, l'objet de la présente invention concerne un conjugué peptidique de formule (I) tel que défini présentement, caractérisé en ce que le groupement A représente un groupement palmitoyle ou un résidu cholestérol.

Lorsque A est une résidu cholestérol, il est entendu que le groupement Y de la formule (II) ci-dessus représente l'atome de carbone de la liaison ester formée entre le cholestérol et X1.

L'objet de la présente invention concerne en outre un conjugué peptidique de formule (I) tel que défini présentement, caractérisé en ce que le sel est un sel d'addition acide, dans lequel l'acide est par exemple l'acide chlorhydrique, l'acide trifluoroacétique et/ou l'acide acétique. De manière préférée, l'acide est choisi pour permettre d'accroître la lipophilicité du conjugué peptidique de formule (I) et ainsi aider sa pénétration dans la peau.

L'objet de la présente invention concerne de plus un conjugué peptidique de formule (I) tel que défini présentement, caractérisé en ce que X₁ est une alanine ou une liaison covalente, et X₂ est une arginine ou une alanine.

L'objet de la présente invention concerne de manière avantageuse un conjugué peptidique de formule (I) tel que défini présentement, caractérisé en ce qu'il est choisi parmi :
- (2) Palm-Ala-Arg-Pro-Ala-Lys-OH,
- (3) Palm-Ala-Arg-Pro-Ala-Lys-NH₂,
- (4) Palm-Ala-Arg-Pro-Ala-Ala-NH₂,
- (5) Palm-Ala-Arg-Ala-Ala-Lys-NH₂,
- (6) Palm-Ala-Ala-Pro-Ala-Lys-NH₂,
- (7) Chol-Ala-Arg-Pro-Ala-Lys-NH₂,
- (9) Palm-Arg-Pro-Ala-Lys-OH,
dans lesquels
- Palm représente un groupement palmitoyle et
- Chol représente le résidu cholestérol.

De manière préférée, l'objet de la présente invention concerne un conjugué peptidique de formule (I) pour son utilisation dans le traitement ou la prévention de l'alopécie, caractérisée en ce que l'alopécie est choisie parmi une alopécie androgénétique, congénitale, acquise, localisée, diffuse, aiguë ou chronique.

L'objet de la présente invention concerne en outre une composition pharmaceutique, dermatologique ou cosmétique comprenant au moins un conjugué peptidique de formule (I) tel que défini présentement caractérisée en outre qu'il s'agit d'une composition à usage topique.

L'objet de la présente invention concerne également une composition telle que définie ci-dessus caractérisée en ce que ladite composition comprend un conjugué peptidique de formule (I) sous forme d'énantiomères et/ou de diastéréoisomères, préférentiellement les acides aminés du conjugué peptidiques étant de forme L ou D exclusivement (i.e. supérieur à un ratio 95/ 5%) ou L/D (i.e. 50/50% ± 10%).

L'objet de la présente invention concerne de plus une composition telle que définie ci-dessus caractérisée en ce qu'il s'agit d'une composition pour le cuir chevelu.

L'objet de la présente invention concerne ainsi une composition telle que définie ci-dessus caractérisée en ce que ladite composition est choisie parmi une lotion, un sérum, un shampooing, tel qu'un shampoing traitant, un spray, un gel ou une crème telle qu'une crème traitante.

L'objet de la présente invention concerne une composition telle que définie ci-dessus caractérisée en ce que ladite composition comprend au moins un peptide de formule (I) tel que décrit ci-dessus à une concentration comprise entre 10⁻⁹ et 10⁻⁴ mole/litre de composition, de préférence entre 10⁻⁷ et 10⁻⁵ mole/litre de composition.

L'objet de la présente invention concerne ainsi l'utilisation cosmétique d'une composition telle que définie ci-dessus pour diminuer la chute des cheveux et/ou stimuler leur pousse.

L'objet de la présente invention concerne également une composition telle que définie ci-dessus, en tant que médicament.

L'objet de la présente invention concerne préférentiellement une composition telle que définie ci-dessus pour son utilisation dermatologique comprenant éventuellement en association avec le conjugué peptidique de formule (I) selon l'invention, un autre principe actif préférentiellement dermatologique tels que ceux définis ci-dessus.

L'objet de la présente invention concerne en outre une composition telle que définie ci-dessus pour son utilisation dans le traitement ou la prévention de l'alopécie.

L'objet de la présente invention concerne une composition telle que définie ci-dessus pour son utilisation ci-dessus, caractérisée en ce que l'alopécie est choisie parmi une alopécie androgénétique, congénitale, acquise, localisée, diffuse, aiguë ou chronique.

### Exemples

Les exemples suivants illustrent la présente invention mais n'en limitent en rien la portée.

### I. Synthèse

La synthèse de peptides en phase solide, selon les exemples suivants, a été réalisée selon une stratégie Fmoc/tBu.

Une quantité connue de résine est introduite dans un réacteur puis des cycles de couplages et de déprotections se succèdent jusqu'à l'obtention du peptide désiré.

Le peptide est ensuite clivé de la résine puis isolé par précipitation puis purifié.

Les peptides ont été synthétisés sur la résine Rink-amide. Les couplages ont été effectués avec l'HBTU comme agent de couplage et la DIEA comme base.

En fin de synthèse, le clivage et la déprotection des chaînes latérales ont été réalisés selon le protocole suivant pour la résine Rink-amide.

### A. Clivage de la résine :

Le clivage de la résine Rink-amide se fait avec du TFA à raison d'environ 10mL/g de résine pendant 1h.

Au bout d'une heure, la résine est filtrée puis rincée avec du DCM. Le filtrat est alors concentré sous vide et le peptide est isolé par précipitation dans l'éther puis centrifugé.

Le surnageant est ensuite éliminé, puis le culot est séché sous vide.

Les composés finaux ont été caractérisés par HPLC analytique et par spectrométrie de masse LC / MS.

Les rendements ont été calculés par rapport à la charge initiale de la résine.

### B. Acylation des peptides :

### P1 : Palmitoylation des peptides

Mettre du DCM dans le réacteur contenant le peptide supporté (le peptide doit être déprotégé du côté N-terminal) et laisser la résine gonfler pendant 15 min.

Solubiliser 3 équivalents de Palm-Cl (équivalents par rapport à la charge de la résine) dans du DCM. Vider ensuite le réacteur et ajouter le Palm-Cl dissout dans le DCM puis agiter pendant 3h.

Laver la résine et sécher sous vide.

### P2 : Greffage du cholestérol sur des peptides

Mettre du DCM dans le réacteur contenant le peptide supporté (le peptide doit être déprotégé du côté N-terminal) et laisser la résine gonfler pendant 15 min. Solubiliser 3 équivalents de cholestérol (équivalents par rapport à la charge de la résine) sous forme de chloroformiate de cholestérol dans du DCM.

Ajouter le Cholestéryl-Cl (synonyme de chloroformiate de cholestérol) dissout dans le DCM puis agiter pendant 3h.

Laver la résine et sécher sous vide.

### • Synthèse du peptide (3) Palm-Ala-Arg-Pro-Ala-Lys-NH₂

Le peptide a été palmitoylé selon le protocole P1 puis le peptide a été clivé de la résine et a été purifié par HPLC préparative. Rendement : 40% ; Pureté : 93% ; LCMS : *m*/*z 779,7* [M+H]⁺

### • Synthèse du peptide (7) Chol-Ala-Arg-Pro-Ala-Lys-NH₂

Le cholestérol a été greffé selon le protocole P2 puis le peptide a été clivé de la résine et purifié par HPLC préparative. Rendement : 50% ; Pureté : 97% ; LCMS : *m*/*z 953,8* [M+H]⁺

### • Synthèse du peptide (5) Palm-Ala-Arg-Ala-Ala-Lys-NH₂

Le peptide a été palmitoylé selon le protocole P1 puis le peptide a été clivé de la résine et purifié par HPLC préparative. Rendement : 60% ; Pureté : 93% ; LCMS : m/z *753,6* [M+H]⁺

### • Synthèse du peptide (6) Palm-Ala-Ala-Pro-Ala-Lys-NH₂

Le peptide a été palmitoylé selon le protocole P1 puis le peptide a été clivé de la résine et purifié par HPLC préparative. Rendement : 75% ; Pureté : 99% ; LCMS : m/z *694,6* [M+H]⁺

### • Synthèse du peptide (9) Palm-Arg-Pro-Ala-Lys-OH

Le peptide a été palmitoylé selon le protocole P1 puis le peptide a été clivé de la résine et purifié par HPCL préparative. Rendement : 60% ; Pureté : 99% ; LCMS : m/z *709,6* [M+H]⁺

Les peptides dont l'utilisation est l'objet de l'invention ont fait l'objet d'essais pharmacologiques permettant de montrer leur activité antichute et repousse des cheveux.

### II. Evaluation de l'activité biologique de 9 peptides

### 1 ∘ Etude de l'effet de peptides sur la prolifération et sur la viabilité des cellules faisant partie du follicule pileux humain

La prolifération cellulaire ainsi que leur viabilité ont été évaluées *in vitro* avec un kit de Promega, *CellTiter-Blue*^{®} *Cell Viability Assay,* qui permet d'estimer le nombre de cellules vivantes présentes dans la culture. Ce kit repose sur la détection, par mesure de fluorescence, de la conversion du colorant la résazurine en un produit fluorescent (la résorufine) par les cellules vivantes et ainsi métaboliquement actives.

L'effet des peptides testés **(1)** et **(9)** aux 4 concentrations différentes (10⁻⁵, 10⁷, 10⁻⁹ et 10⁻¹¹M) a été examiné sur cinq types de cellules : kératinocytes humains cutanés immortalisés, HaCaT ; kératinocytes folliculaires humains, HHFK (*Human Hair Follicular Keratinocytes*) ; fibrobroblastes dermiques humains, NHDF (*Normal Human Dermal Fibroblastes*) ; cellules de papille dermique du follicule pileux humain, HFDPC (*Hair Follicle Dermal Papilla Cell*) et mélanocytes épidermiques humains, NHEM (Normal Human Epidermal Melanocytes) exposées à la molécule étudiée pendant 72 heures.

Les résultats obtenus montrent que tous les peptides étudiés aux 4 concentrations testées ne présentent aucune toxicité vis-à-vis des quatre types des cellules utilisées dans cette expérience.

D'autre part, une légère stimulation de la prolifération de NHDF par **(6)** à 10⁻⁵M (+11%) et de HaCaT par **(1)** à 10⁻⁵M (+15%) a été observée.

### 2 ° Analyse par PCR quantitative (TLDA) de l'expression des gènes codant pour les facteurs protéiques potentiellement impliqués dans l'alopécie androgénétique masculine

Pour étudier l'effet de 9 peptides sur ces gènes, les cellules folliculaires HFDPC ont été traitées pendant 24 heures avec des molécules testées aux concentrations 10⁻⁷M et 10⁻⁹M.

Les ARN ont été extraits et purifié à partir des culots cellulaires en utilisant le Kit RNAXS (Macherey-Nagel). La quantification des ARN a été réalisée au Nanodrop et la qualité des échantillons a été vérifiée sur Bioanalyzer avec les puces RNA6000nano (Agilent). Les cDNA ont été synthétisés à partir de 300 ng d'ARN en utilisant le Kit High Capacity cDNA Reverse Transcription (LifeTech). Les QPCR de contrôle qualité des cDNA ont été réalisées en microplaque sur le gène de référence GAPDH et le gène SIRT1.

Les expériences finales de QPCR ont été réalisées dans un volume de 1µl sur des cartes microfluidiques (TaqMan Low Density Arrays, LifeTech) et l'appareil de QPCR ABI 7900HT. Les réactions d'amplification ont été faites avec duplicata technique, en utilisant 1 à 2 ng de cDNA par réaction de QPCR et le Mix TaqMan Universel Master Mix II (LifeTech).

Les valeurs de Ct ont été obtenues en utilisant le logiciel RQ Manager. Pour les analyses des résultats de QPCR, les valeurs de Ct ont été limitées à 35 cycles et les duplicatas techniques sont moyennés. Le logiciel GenexPro a été utilisé pour les études de stabilité et de sélection des meilleurs gènes de référence. Les variations d'expression sont calculées par la méthode de quantification relative et exprimées en pourcentage d'augmentation de l'expression du gène d'intérêt par rapport aux contrôles non traités.

Les résultats, présentés dans le Tableaux 1, montrent que **(1), (2), (3), (5), (6)** et **(9)** stimulent plusieurs gènes dont l'expression est réduite chez les sujets caractérisés par l'alopécie. L'intensité de l'effet varie en fonction de la structure du peptide testé et de sa concentration.

**Tableau 1 : Analyse par qPCR de l'effet in vitro des peptides (10⁻⁵M) sur la modification de l'expression des gènes codant pour les facteurs de croissance impliqués dans la pousse de cheveux**

| | | **% stimulation** | | | | | |
|---|---|---|---|---|---|---|---|
| | **Formules** | **KGF** | **HGF** | **PDG F** | **VEGF** | **IGF1** | **BMP2** |
| **1** | H-Ala-Arg-Pro-Ala-Lys-OH (exemple hors invention) | 14% | | | | 32% (10⁻⁷) | 26% (10⁻⁹) |
| **2** | Palm-Ala-Arg-Pro-Ala-His-OH | 71% | | 29% | 11% | 136% (10⁻⁹) | |
| **3** | Palm-Ala-Arg-Pro-Ala-Lys-NH₂ | | | | 83% | | |
| **4** | Palm-Ala-Arg-Pro-Ala-Ala-NH₂ | | | | | | |
| **5** | Palm-Ala-Arg-Ala-Ala-Lys-NH₂ | | | | 10% | 29% (10⁻⁷) | |
| | | | | | | 16% (10⁻⁹) | |
| **6** | Palm-Ala-Ala-Pro-Ala-Lys-NH₂ | | | | 24% | 94% (10⁻⁹) | 116% (10⁻⁹) |
| **7** | Chol-Ala-Arg-Pro-Ala-Lys-NH₂ | | 49% | | | | |
| **8** | H-Arg-Pro-Lys-OH (exemple hors invention) | 14% | | | | | |
| **9** | Palm-Arg-Pro-Ala-Lys-OH | 156% | | 304% | 93% | | |

| | | | | | | | |
|---|---|---|---|---|---|---|---|
| Etude réalisée sur les cellules HFDPc | | | | | | | |

### 3 ° Etude de l'effet de peptides sur la sécrétion de facteurs de croissance impliqués dans la régulation du cycle pilaire

Compte tenu du fait que la pousse des cheveux reste sous contrôle de nombreux facteurs de croissance dont le rôle est bien établi, le pouvoir des peptides à induire la sécrétion de ces facteurs par des cellules des cheveux a été évalué. D'autre part, il a été intéressant à savoir si ces peptides ont un effet sur la sécrétion des facteurs protéiques codés par les gènes réprimés dans l'alopécie et dont l'expression a été augmentée suite aux traitements avec des composés testés.

Les cellules NHDF, HFDPC, HHFK et NHEM ont été traitées avec des peptides étudiés pendant 48h. A la fin du traitement les surnageants cellulaires ont été récupérés et conservé à -80°C jusqu'à l'analyse. Les facteurs d'intérêt secrétées dans le surnageant ont été dosées par ELISA^{®} (R&D) ou BioPlex^{®} (BioRad^{®}).

Les résultats obtenus (Tableau 2) ont mis en évidence un effet stimulateur de peptides aux concentrations de (10⁻⁷M et 10⁻⁵M) sur la sécrétion de plusieurs facteurs de croissance impliqués dans la pousse des cheveux, tels que PDGF, KGF, VEGF, HGF, SCF, NGF. D'autre part, le traitement des cellules faisant partie du follicule pileux conduit à une augmentation de la sécrétion des facteurs protéiques codés par des gènes dont l'expression est réduite chez des sujets développant une alopécie (BMP2). **(6)** aussi bien que **(1)** manifestent l'activité la plus significative.

Les résultats sont exprimés en pourcentage d'augmentation de la concentration du facteur présent dans le surnageant par rapport au contrôle non traité. L'intensité de l'effet varie en fonction de la concentration du peptide étudié et du type de cellules traitées.

**Tableau 2. Effet in vitro de peptides sur la sécrétion des facteurs impliqués dans la pousse des cheveux par des cellules du follicule pileux**

| **Peptide** | **[M]** | **HFPDC** | **NHDF** | **HHKF** | **NHEM** |
|---|---|---|---|---|---|
| | 10⁻⁷ | **PDGF 38%** | NGF 12% | HGF 52% | VEGF 15% |
| | | KGF 13% | VGF 407% | SCF 9% | |
| | | | | NGF 10% | |
| **(6)** | 10⁻⁵ | **PDGF 15%** | VEGF 40%, 275% | | |
| | | KGF 7% | | NT | NT |
| | | VEGF 18% | NGF 51% | | |
| | | BMP2 44% | SCF 8% | | |
| (1) (exemple hors invention) | 10⁻⁷ | PDGF 31% | VEGF 4% | NT non testé | |
| | | KGF 8% | | | |
| | | VEGF 9% | | | |
| | 10⁻⁹ | PDGF 23% | VEGF5% | | |
| | | KFG 25% | HGF 18% | | |
| | | VEGF 4% | NGF 13% | | |
| | | BMP2 22% | | | |

| **Peptide** | **[M]** | **HFPDC** | **NHDF** | **HHKF** | **NHEM** |
|---|---|---|---|---|---|
| (5) | 10⁻⁷ | **PDGF 55%** | HGF 21% | HGF 104% | VEGF 5% |
| | | | | | HGF 8% |
| | 10⁻⁵ | **PDGF 39%** | VEGF 12% | NT | NT |
| | | | NGF 15% | | |
| (3) | 10⁻⁷ | KGF 53% | NT | | |
| | | VEGF 9% | | | |
| | 10⁻⁹ | **PDGF 39%** | VEGF 48% | | |
| | | KFG 45% | HGF 5% | | |
| | | HGF 33% | NGF 89% | | |
| | | NGF 6% | | | |
| **(7)** | 10⁻⁷ | NT | HGF 104% | | |
| | | | SCF 10% | | |
| | | | NGF 11% | | |
| | 10⁻⁹ | NT | NT | | |
| **(4)** | 10⁻⁷ | **PDGF 8%** | HGF 188% | | |
| | | | VEGF 9% | | |
| | | | SCF 6% | | |
| | | | NGF 12% | | |
| | 10⁻⁹ | NT | HGF 95% | | |
| | | | SCF 8% | | |
| | | | NGF 18% | | |

| **Peptide** | **[M]** | **HFPDC** | **NHDF** | | **HHKF** |
|---|---|---|---|---|---|
| **(9)** | 10⁻⁷ | **PDGF 117%** | SCF 8% | | HGF 94% |
| | | | | | SCF 11% |
| | 10⁻⁵ | **PDGF 62%** | VEGF 24% | | |
| | | VEGF 10% | | | |
| **(2)** | 10⁻⁷ | HGF 565% | HGF 13% | HGF 28% | |
| | | | SCF 37% | | |
| | 10⁻⁵ | HGF 403% | SCF 24% | | |

| **Peptide** | **[M]** | **HFPDC** | **NHDF** | | |
|---|---|---|---|---|---|
| **(8)** (exemple hors invention) | 10⁻⁷ | HGF 129% | | | |
| | 10⁻⁵ | HGF 26% | HGF 8% | | |

### III. Etude de l'activité stimulante de (1), (6) et (9) sur la croissance de cheveux isolés ex vivo, et sur les changements morphologiques associés

Cette étude a été réalisée sur cheveux microdisséqués à partir de plasties de cuir chevelu provenant de femmes de 52 à 71 ans. Les cheveux isolés ont été mis en survie pendant 11 à 12 jours dans du milieu Williams, dans des conditions de culture cellulaire classiques (37°C, 5% CO₂). Le peptide est incorporé au milieu de culture à deux concentrations : 10⁻⁷M et 10⁻⁹M, tous les 2 jours. Le Minoxidil^{®} (2,4-diamino 6-pipéridinopyrimidine 3-oxyde) sulfate a été utilisé comme contrôle positif à la concentration 10⁻⁵ M.

Pour évaluer l'effet de **(1), (6)** ou **(9)** sur la pousse des cheveux, les cheveux ont été photographiés et leurs longueurs mesurées à l'aide d'un logiciel d'analyse d'image. Ainsi la croissance des cheveux entre J0 et J11/J12 a pu être calculée et comparée à celle du témoin non traité. A la fin des traitements (J11 ou J12), les cheveux ont été prélevés, congelés ou fixés pour des immunomarquages et des colorations histologiques.

Les résultats obtenus, résumés dans le Tableau 3, montrent que les peptides **(1)** et **(6),** et plus faiblement **(9),** stimulent la pousse des cheveux après 11 à 12 jours de traitement *ex vivo.*

L'augmentation de la croissance avec **(1)** à 10⁻⁹M est proche de celle obtenue après traitement avec le minoxidil sulfate (+48% à J11). L'augmentation de la croissance avec **(6)** à 10⁻⁹M est supérieure à celle obtenue après traitement avec le minoxidil sulfate (+28% à J12).

D'autre part, ces trois peptides stimulent l'expression des marqueurs folliculaires impliqués dans la régulation des cellules souches (CK15, CK19), dans la croissance folliculaire (IGF1), et dans l'ancrage du cheveu à la matrice extracellulaire (collagène IV, laminine-5).

**Tableau 3. Effet ex vivo de 9 peptides sur la pousse de cheveux**

| **Vs Témoin** | **(1) (exemple hors invention)** | | **(6)** | | **(9)** | |
|---|---|---|---|---|---|---|
| | **10⁻⁷M** | **10⁻⁹M** | **10⁻⁷M** | **10⁻⁹M** | **10⁻⁷M** | **10⁻⁹M** |
| **Croissance à J11/J12** | Pas d'effet | +40% | +28% | +38%^{#} | Pas d'effet | +5% |
| **Marqueurs folliculaires** | ↗ IGF1 Laminine-5 | ↗ CK19 | | | ↗ CK19 | ↗ CK19 |
| | | CD34 | | | CK15 | CK15 |
| | Coll IV | Coll IV | | | | |

| | | | | | | |
|---|---|---|---|---|---|---|
| *↗ augmentation ; ^{#} Student t-test significatif avec p<0.1.* | | | | | | |

Cette invention concerne des stimulants de la pousse des cheveux renfermant comme principe actif des peptides synthétiques de la formule générale A-X₁-X₂-Pro-Ala-X-B. L'ensemble des observations obtenues dans les conditions opératoires de cette étude identifie ces peptides comme des activateurs de l'expression génique réprimée dans le cas de l'alopécie ainsi comme des stimulateurs de la production des facteurs de croissance essentiels pour la pousse des cheveux.

L'évaluation *ex vivo* de l'activité biologique met en évidence leur effet inducteur de la croissance des cheveux. Les peptides étudiés induisent l'ensemble des modifications décrites aux concentrations qui varient entre 10⁻⁹M et 10⁻⁵M. Ainsi, la préparation selon l'invention peut être efficacement employée au titre de préparation à usage externe, telle qu'un produit pharmaceutique ou cosmétique pour favoriser la croissance des cheveux et/ou d'augmenter leur densité et/ou de diminuer leur chute.

Les exemples de formulation suivants illustrent, par ailleurs, la présente invention :
**Exemple 1** : lotion comprenant le peptide (1) (peptide hors invention)

| | **En g** |
|---|---|
| - Peptide (1) | 5.10⁻⁶ |
| - Ethanol à 95° | 20 |
| - Propylène glycol | 10 |
| - Eau conservateurs | qsp 100 |

**Exemple 2** : lotion comprenant le peptide **(9)**

| | **En g** |
|---|---|
| - Peptide | 10⁻⁵ |
| - Eau | 81 |
| - Keltrol T | 0,5 |
| - Techpolymer MB - 4°C | 1 |
| - Sepigel 305 | 0,5 |
| - Huile de Silicone 140 | 2 |
| - Butylène Glycol | 5 |

## Revendications

1. Conjugué peptidique de formule (I) :
A-X₁-X₂-Pro-Ala-X-B (I)
dans lequel:
- A représente un groupement acyle en C₆ à C₂₀ ou un résidu cholestérol;
- X₁ représente une liaison covalente, une alanine ou une proline ;
- X₂ représente une arginine, une lysine, ou une alanine ;
- X représente une lysine, une alanine, ou une phénylalanine ; et
- B représente un hydroxyle ou une amine ;
ou l'un de ses sels, préférentiellement pharmaceutiquement, dermatologiquement ou cosmétiquement acceptables.

2. Conjugué peptidique de formule (I) selon la revendication 1, **caractérisé en ce que** le groupement A représente un groupement acyle en C₁₀ à C₂₀, préférentiellement un groupement palmitoyle, ou un résidu de cholestérol selon la formule (II) : 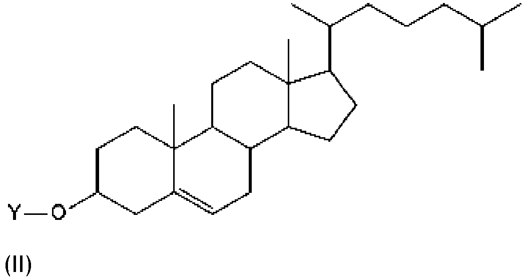 dans laquelle :
- Y représente l'atome de carbone de la liaison ester entre le résidu cholestérol et X1..

3. Conjugué peptidique de formule (I) selon la revendication 1 ou 2, **caractérisé en ce que** le sel est un sel d'addition acide, dans lequel l'acide est par exemple l'acide chlorhydrique, l'acide trifluoroacétique et/ou l'acide acétique.

4. Conjugué peptidique de formule (I) selon l'une quelconque des revendications 1 à 3, **caractérisé en ce que** X₁ est une alanine ou une liaison covalente, et X₂ est une arginine ou une alanine.

5. Conjugué peptidique de formule (I) selon l'une quelconque des revendications 1 à 4, **caractérisé en ce qu'**il est choisi parmi :
- (2) Palm-Ala-Arg-Pro-Ala-Lys-OH,
- (3) Palm-Ala-Arg-Pro-Ala-Lys-NH₂,
- (4) Palm-Ala-Arg-Pro-Ala-Ala-NH₂,
- (6) Palm-Ala-Ala-Pro-Ala-Lys-NH₂,
- (7) Chol-Ala-Arg-Pro-Ala-Lys-NH₂,
- (9) Palm-Arg-Pro-Ala-Lys-OH,
dans lesquels
- Palm représente un groupement palmitoyle et
- Chol représente un résidu cholestérol.

6. Procédé de fabrication du conjugué peptidique de formule (I) selon l'une quelconque des revendications 1 à 5, **caractérisé en ce que** ledit procédé comprend les étapes successives suivantes :
a. synthèse, au moins partiellement sur support solide, du brin peptidique de formule H-X₁-X₂-Pro-Ala-X-B, dans lequel
- X₁, X₂, X sont tels que définis selon l'une quelconque des revendications 1 à 5 et sont convenablement protégés ;
- B est le support solide ou un groupement OH ou NH₂ convenablement protégé ;
b. greffage du groupement A au brin peptidique de l'étape (a) par réaction d'acylation, préférentiellement par l'utilisation d'un acyle activé en présence d'une base;
c. déprotection des acides aminés protégés éventuellement conjointement au clivage du peptide de la résine, lorsque B est un support solide;
d. éventuelle purification du conjugué peptidique de formule (I) obtenu, par exemple par HPLC ; et
e. récupération du produit de formule (I).

7. Conjugué peptidique de formule (I) selon l'une quelconque des revendications 1 à 5, en tant que médicament.

8. Conjugué peptidique de formule (I) selon l'une quelconque des revendications 1 à 5, pour son utilisation dermatologique éventuellement en association avec un autre principe actif préférentiellement dermatologique.

9. Conjugué peptidique de formule (I) selon l'une quelconque des revendications 1 à 5, pour son utilisation dans le traitement ou la prévention de l'alopécie.

10. Conjugué peptidique de formule (I) selon l'une quelconque des revendications 1 à 5 pour son utilisation selon la revendication 9, **caractérisée en ce que** l'alopécie est choisie parmi une alopécie androgénétique, congénitale, acquise, localisée, diffuse, aiguë ou chronique.

11. Composition pharmaceutique, dermatologique ou cosmétique comprenant au moins un conjugué peptidique de formule (I) selon l'une quelconque des revendications 1 à 5 et 8 à 10

12. Composition selon la revendication 11 caractérisée en outre qu'il s'agit d'une composition à usage topique.

13. Composition selon la revendication 11 ou 12 **caractérisée en ce que** ladite composition comprend un conjugué peptidique de formule (I) sous forme d'énantiomères et/ou de diastéréoisomères, préférentiellement les acides aminés du conjugué peptidiques étant de forme L ou D exclusivement ou L/D.

14. Composition selon l'une quelconque des revendications 11 à 13 **caractérisée en ce qu'**il s'agit d'une composition pour le cuir chevelu.

15. Composition selon l'une quelconque des revendications 11 à 14 **caractérisée en ce que** ladite composition est choisie parmi une lotion, un sérum, un shampooing, tel qu'un shampoing traitant, un spray, un gel ou une crème telle qu'une crème traitante.

16. Composition selon l'une quelconque des revendications 11 à 15 **caractérisée en ce que** ladite composition comprend au moins un peptide de formule (I) selon l'une quelconque des revendications 1 à 5 à une concentration comprise entre 10⁻⁹ et 10⁻⁴ mole/litre de composition, de préférence entre 10⁻⁷ et 10⁻⁵ mole/litre de composition.

## Patentansprüche

1. Peptidkonjugat mit der Formel (I):
A-X₁-X₂-Pro-Ala-X-B (I)
wobei:
- A eine C₆- bis C₂₀-Acyl-Gruppe oder einen Cholesterinrest darstellt;
- X₁ eine kovalente Bindung, ein Alanin oder ein Prolin darstellt;
- X₂ ein Arginin, ein Lysin oder ein Alanin darstellt;
- X ein Lysin, ein Alanin oder ein Phenylalanin darstellt; und
- B ein Hydroxyl oder ein Amin darstellt;
oder eines seiner Salze, die vorzugsweise pharmazeutisch, dermatologisch oder kosmetisch annehmbar sind.

2. Peptidkonjugat mit der Formel (I) nach Anspruch 1, **dadurch gekennzeichnet, dass** die Gruppe A eine C₁₀-bis C₂₀-Acyl-Guppe, vorzugsweise eine Palmitoyl-Gruppe, oder einen Cholesterinrest nach der Formel (II) darstellt: 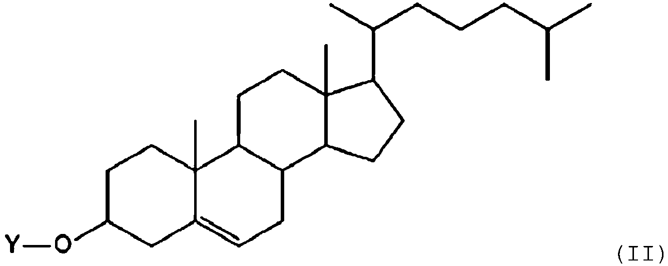 wobei:
- Y das Kohlenstoffatom der Esterverbindung zwischen dem Cholesterinrest und X1 darstellt.

3. Peptidkonjugat mit der Formel (I) nach Anspruch 1 oder 2, **dadurch gekennzeichnet, dass** das Salz ein Säure-Additionssalz ist, wobei die Säure zum Beispiel Salzsäure, Trifluoressigsäure und/oder Essigsäure ist.

4. Peptidkonjugat mit der Formel (I) nach einem der Ansprüche 1 bis 3, **dadurch gekennzeichnet, dass** X₁ ein Alanin oder eine kovalente Bindung ist und X₂ ein Arginin oder ein Alanin ist.

5. Peptidkonjugat mit der Formel (I) nach einem der Ansprüche 1 bis 4, **dadurch gekennzeichnet, dass** es ausgewählt ist aus:
- (2) Palm-Ala-Arg-Pro-Ala-Lys-OH,
- (3) Palm-Ala-Arg-Pro-Ala-Lys-NH₂,
- (4) Palm-Ala-Arg-Pro-Ala-Ala-NH₂,
- (6) Palm-Ala-Ala-Pro-Ala-Lys-NH₂,
- (7) Chol-Ala-Arg-Pro-Ala-Lys-NH₂,
- (9) Palm-Arg-Pro-Ala-Lys-OH,
wobei:
- Palm eine Palmitoyl-Gruppe darstellt und
- Chol einen Cholesterinrest darstellt.

6. Verfahren zur Herstellung eines Peptidkonjugats mit der Formel (1) nach einem der Ansprüche 1 bis 5, **dadurch gekennzeichnet, dass** das Verfahren die folgenden aufeinanderfolgenden Schritte umfasst:
a. Synthese, zumindest teilweise auf einem festen Träger, des Peptidstrangs mit der Formel H-X₁-X₂-Pro-Ala-X-B, wobei
- X₁, X₂, X nach einem der Ansprüche 1 bis 5 sind und in geeigneter Weise geschützt sind;
- B der feste Träger oder eine in geeigneter Weise geschützte OH- oder NH₂-Gruppe ist;
b. Grafting der Gruppe A an den Peptidstrang aus dem Schritt (a) durch Acylierungsreaktion, vorzugsweise durch die Nutzung eines bei Vorhandensein einer Base aktivierten Acyls;
c. Entschützen der geschützten Aminosäuren, gegebenenfalls gemeinsam mit der Spaltung des Peptids von dem Harz, wenn B ein fester Träger ist;
d. gegebenenfalls Reinigen des erhaltenen Peptidkonjugats mit der Formel (I), zum Beispiel durch HPLC; und
e. Rückgewinnen des Produkts mit der Formel (I).

7. Peptidkonjugat mit der Formel (I) nach einem der Ansprüche 1 bis 5 als Arzneimittel.

8. Peptidkonjugat mit der Formel (I) nach einem der Ansprüche 1 bis 5 zur dermatologischen Verwendung, gegebenenfalls in Verbindung mit einem anderen, vorzugsweise dermatologischen, Wirkstoff.

9. Peptidkonjugat mit der Formel (I) nach einem der Ansprüche 1 bis 5 zur Verwendung bei der Behandlung oder der Prävention von Alopezie.

10. Peptidkonjugat mit der Formel (I) nach einem der Ansprüche 1 bis 5 zur Verwendung nach Anspruch 9, **dadurch gekennzeichnet, dass** die Alopezie aus einer androgenetischen, angeborenen, erworbenen, lokalisierten, diffusen, akuten oder chronischen Alopezie gewählt ist.

11. Pharmazeutische, dermatologische oder kosmetische Zusammensetzung, die mindestens ein Peptidkonjugat mit der Formel (I) nach einem der Ansprüche 1 bis 5 und 8 bis 10 umfasst.

12. Zusammensetzung nach Anspruch 11, ferner **dadurch gekennzeichnet, dass** es sich um eine Zusammensetzung zur topischen Verwendung handelt.

13. Zusammensetzung nach Anspruch 11 oder 12, **dadurch gekennzeichnet, dass** die Zusammensetzung ein Peptidkonjugat mit der Formel (I) in der Form von Enantiomeren und/oder Diastereoisomeren umfasst, wobei die Aminosäuren des Peptidkonjugats vorzugsweise ausschließlich L- oder D-förmig oder L/D-förmig sind.

14. Zusammensetzung nach einem der Ansprüche 11 bis 13, **dadurch gekennzeichnet, dass** es sich um eine Zusammensetzung für die Kopfhaut handelt.

15. Zusammensetzung nach einem der Ansprüche 11 bis 14, **dadurch gekennzeichnet, dass** die Zusammensetzung ausgewählt ist aus einer Lotion, einem Serum, einem Shampoo wie einem Behandlungsshampoo, einem Spray, einem Gel oder einer Creme wie einer Behandlungscreme.

16. Zusammensetzung nach einem der Ansprüche 11 bis 15, **dadurch gekennzeichnet, dass** die Zusammensetzung mindestens ein Peptid mit der Formel (I) nach einem der Ansprüche 1 bis 5 mit einer Konzentration zwischen 10⁻⁹ und 10⁻⁴ Mol/Liter Zusammensetzung, vorzugsweise zwischen 10⁻⁷ und 10⁻⁵ Mol/Liter Zusammensetzung, umfasst.

## Claims

1. A peptide conjugate of formula (I):
A-X₁-X₂-Pro-Ala-X-B (I)
wherein:
- A represents a C₆ to C₂₀ acyl group or a cholesterol residue;
- X₁ represents a covalent bond, an alanine or a proline;
- X₂ represents an arginine, a lysine, or an alanine;
- X represents a lysine, an alanine, or a phenylalanine; and
- B represents a hydroxyl or an amine;
or one of its salts, preferentially pharmaceutically, dermatologically or cosmetically acceptable salts.

2. Peptide conjugate of formula (I) according to claim 1, wherein group A represents a C₁₀ to C₂₀ acyl group, preferentially a palmitoyl group, or a cholesterol residue according to formula (II): 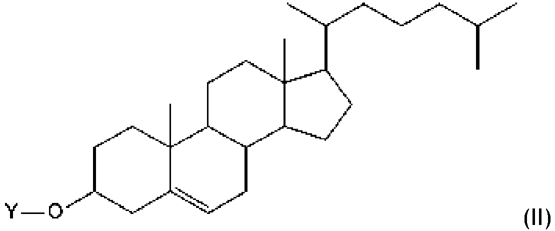 wherein:
- Y represents the carbon atom of the ester bond between the cholesterol residue and X₁.

3. Peptide conjugate of formula (I) according to claim 1 or 2, wherein the salt is an acid addition salt, wherein the acid is for example hydrochloric acid, trifluoroacetic acid and/or acetic acid.

4. Peptide conjugate of formula (I) according to any one of claims 1 to 3, wherein X₁ is an alanine or a covalent bond, and X₂ is an arginine or an alanine.

5. Peptide conjugate of formula (I) according to any one of claims 1 to 4,
wherein the peptide conjugate of formula (I) is selected from:
- (2) Palm-Ala-Arg-Pro-Ala-Lys-OH,
- (3) Palm-Ala-Arg-Pro-Ala-Lys-NH₂,
- (4) Palm-Ala-Arg-Pro-Ala-Ala-NH₂,
- (6) Palm-Ala-Ala-Pro-Ala-Lys-NH₂,
- (7) Chol-Ala-Arg-Pro-Ala-Lys-NH₂,
- (9) Palm-Arg-Pro-Ala-Lys-OH,
and wherein
- Palm represents a palmitoyl group and
- Chol represents a cholesterol residue.

6. A process for manufacturing the peptide conjugate of formula (I) according to claim 1, wherein said process comprises the following successive steps:
a. synthesis, at least partially on solid support, of the peptide strand of formula H-X₁-X₂-Pro-Ala-X-B, wherein
- X₁, X₂, X are as defined in any one of claims 1 to 5 and are suitably protected;
- B is the solid support or a suitably protected OH or NH₂ group;
b. grafting of group A onto the peptide strand of step (a) by acylation reaction, preferentially by the use of an activated acyl in the presence of a base;
c. deprotection of the protected amino acids optionally together with cleavage of the peptide from the resin, when B is a solid support;
d. optional purification of the peptide conjugate of formula (I) obtained, for example by HPLC; and
e. collection of the product of formula (I).

7. Peptide conjugate of formula (I) according to any one of claims 1 to 5, as drug.

8. Peptide conjugate of formula (I) according to any one of claims 1 to 5, for dermatological use preferably in combination with another dermatological active ingredient.

9. Peptide conjugate of formula (I) according to any one of claims 1 to 5, for use in the treatment or the prevention of alopecia.

10. Peptide conjugate of formula (I) according to any one of claims 1 to 5 for use according to claim 9, wherein alopecia is selected from androgenetic, congenital, acquired, localized, diffuse, acute or chronic alopecia.

11. Pharmaceutical, dermatological or cosmetic composition comprising at least one peptide conjugate of formula (I) according to any one of claims 1 to 5 and 8 to 10.

12. Composition according to claim 11, further **characterized in that** it is a composition for topical use.

13. Composition according to claims 11 or 12, wherein said composition comprises the peptide conjugate of formula (I) in the form of enantiomers and/or of diastereoisomers, preferentially the amino acids of the peptide conjugate being of L or D form exclusively or of L/D form.

14. Composition according to any one of claims 11 to 13, **characterized in that** the composition is for the scalp.

15. Composition according to any one of claims 11 to 14, **characterized in that** said composition is chosen from the group consisting of a lotion, a serum, a shampoo, such as a medicated shampoo, a spray, a gel, or a cream such as a treating cream.

16. Composition according to any one of claims 11 to 15 **characterized in that** said composition comprises at least one peptide conjugate of formula (I) according to any one of claims 1 to 5 at a concentration ranging from 10⁻⁹ to 10⁻⁴ mole/litre of composition, preferably from 10⁻⁷ to 10⁻⁵ mole/litre of composition.
